Europäisches Patentamt

(19) European Patent Office

Office européen des brevets

(11) Publication number : **0 242 985 B1**

# EUROPEAN PATENT SPECIFICATION

(45) Date of publication of patent specification :
**05.02.92 Bulletin 92/06**

(51) Int. Cl.[5] : **A61M 25/00**

(21) Application number : **87302366.7**

(22) Date of filing : **19.03.87**

(54) **Preformable catheter assembly and stylet therefor.**

(30) Priority : **26.03.86 US 844397**

(43) Date of publication of application :
**28.10.87 Bulletin 87/44**

(45) Publication of the grant of the patent :
**05.02.92 Bulletin 92/06**

(84) Designated Contracting States :
**AT BE CH DE ES FR GB IT LI LU NL SE**

(56) References cited :
**DE-A- 3 100 546**
**US-A- 3 419 010**
**US-A- 3 923 066**
**US-A- 3 957 055**
**US-A- 4 185 639**
**US-A- 4 504 268**

(73) Proprietor : **SHERWOOD MEDICAL COMPANY**
**1915 Olive Street**
**St. Louis, Missuri 63103 (US)**

(72) Inventor : **Banning, Robert Dean**
**5 Shadow Ridge Drive**
**St.Peters Missouri 63376 (US)**
Inventor : **Crouther, Ronald**
**2382 Capitol Landing Drive**
**Chesterfield Missouri 63017 (US)**
Inventor : **Davison, Thomas Walter**
**83 Farm Hill Road**
**North Attleboro, Massachusetts 02760 (US)**

(74) Representative : **Porter, Graham Ronald**
**c/o Wyeth Laboratories Huntercombe Lane**
**South**
**Taplow Maidenhead Berkshire SL6 0PH (GB)**

EP 0 242 985 B1

## Description

This invention relates to a preformable catheter assembly and a malleable stylet therefor.

Cardiopulmonary bypass vascular catheters, for example, left atrial or left heart vent and left ventricular vent catheters, are used to drain fluid from the left ventricle to prevent excessive pressure build-up in the left heart portion during bypass surgery. Such catheters are often inserted with the aid of a stylet and may be preformable to a desired profile. The left atrial vent catheter may be inserted through the right superior pulmonary vein, left atrium, and mitral valve, and into the left ventricle. The left ventricular vent catheter may be introduced directly into the left ventricle through the ventricle wall. After insertion, the stylet is removed from the catheter and the catheter is connected to an extracorporeal system that includes artificial heart and lung apparatus.

Hitherto many such catheters have been made of polyvinyl chloride (PVC) for use with a rigid plastic stylet or with a malleable wire embedded in a sidewall of the catheter. However, since heart surgery is now being performed at relatively low temperatures, conventional PVC catheters have become less desirable because they become relatively rigid and less flexible at the lower temperatures making the catheter more difficult to manipulate during insertion and removal from the patient. Also, with relatively stiff catheters there is greater risk of damage to the heart during the operation. For this reason, such catheters have more recently been made of silicone rubber which is soft and supple, and these characteristics are substantially unaffected by the low temperatures encountered during surgery. Because silicone rubber catheters are soft and supple, there is less chance of damage to the patient during insertion and removal of the catheter as well as during the operation.

If the diameter of the stylet is excessivley small, it may kink and bend in the catheter making the manual preshaping of the catheter less accurate or controllable. Stylets have been formed of closely coiled stainless steel wire so that the stylet has a substantially larger diameter than that of a straight wire in order to more nearly fill the catheter lumen. In this way, the stylet can have a sufficiently large diameter relative to the catheter lumen so as to produce a catheter having sufficiently good handling and shaping characteristics, and yet have a high malleability due to the small diameter of wire used in the coiled stylet. However, coiled stainless steel wire stylets are relatively heavy and expensive compared to straight wire stylets.

Catheters having a malleable wire embedded in the sidewall of the catheter have been used to allow shaping of the catheter prior to insertion but the suppleness or flexibility of such catheters while in the heart and vessels of the patient are limited by the presence of the wire which cannot be removed from the catheter. Non-malleable plastic such as nylon or high density polyethylene rods have been proposed as stiffeners but are generally limited to catheters that do not require manual preshaping.

US-A-3,957 055 discloses a guide in stylet form for aiding the insertion of catheters into the body, characterised by a core of solid, flexible metal completely encased in a self-lubricating material and an adjustable stop which may also be used as a grip for manipulation of the guide and an anchor for forming a handle by an end of the guide. The cover for the core is a coating of a polymer material moulded or sprayed onto the core. Such coatings may crack during twisting or bending of the core when the stylet is in use.

It is therefore an object of the present invention to provide a malleable catheter assembly having a removable stylet which overcomes the aforementioned problems.

According to the invention there is provided a catheter and stylet assembly comprising a tube of flexible material adapted for insertion into a patient and having a lumen extending therein, and a stylet assembly removably insertable into said lumen, the stylet including only one malleable metal wire, characterised by tubular cover means surrounding and covering at least a major portion of said wire including a portion adjacent the distal end of said wire, and a handle connected to proximal end portions of said wire and said tubular cover means, said tubular cover means having an inner diameter sufficiently greater than the outer diameter of said wire so as to allow insertion of said wire into said tubular cover means during assembly of said stylet.

Such a cover increases the effective diameter of the stylet without substantially affecting the malleability of the wire. The cross-sectional area of the cover may be greater than the cross-sectional area of the wire. Preferably the cover is a tubular extrusion or sleeve into which the wire is inserted; the distal end of the sleeve is closed by, for example, heat sealing.

In a preferred embodiment the wire is of aluminium and the cover is of polypropylene. The outer diameter of the stylet is preferably at least 60% of the diameter of the catheter lumen.

The handle with the cover preferably completely encases the wire.

The catheter may be of silicone rubber. Releasable latching means may be provided between a handle of the stylet and the proximal end of the catheter to hold the stylet in the catheter during the insertion procedure.

The latching means preferably comprise hook means on the handle which releasably engage a flange of a connector for the catheter. The hook means may be a flexible arm moulded integrally with said handle.

Other features of the invention will be apparent from the following description of a preferred embodiment shown by way of example only in the accompanying drawing in which:-

Figure 1 is a longitudinal cross-section through a cardiopulmonary left atrial vent catheter assembly in accordance with the present invention;

Figure 2 is an enlarged cross-section taken along line 2-2 of Figure 1;

Figure 3 is a longitudinal cross-section through the stylet of Figure 1; and

Figure 4 is a side elevation on a slightly reduced scale of the catheter assembly of Figure 1 but after it has been manually formed into a curved configuration.

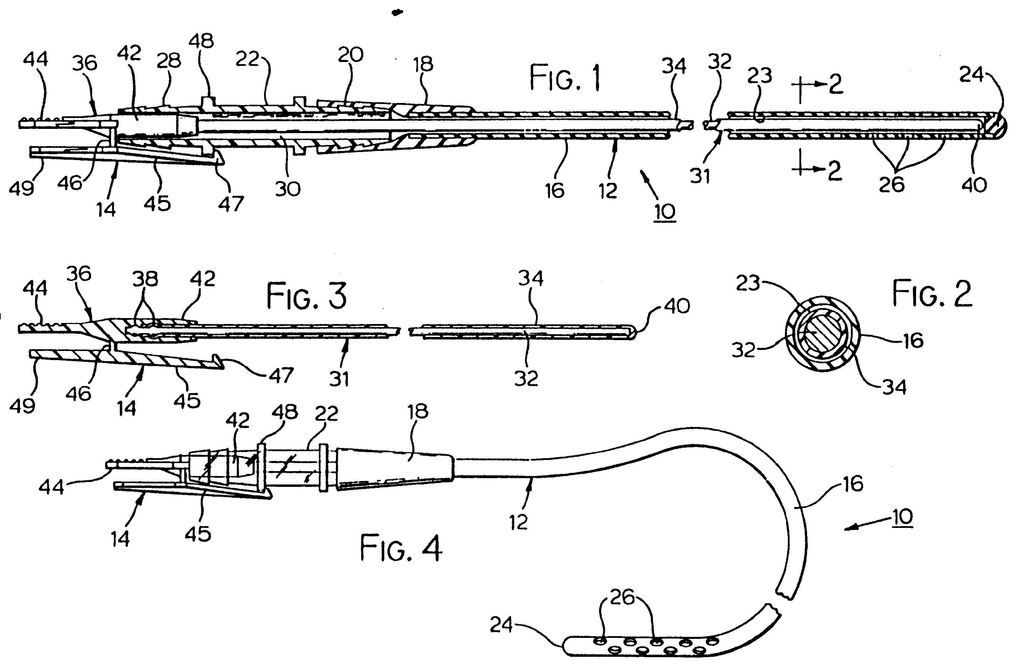

Referring now to the drawings, and particularly to Figures 1 and 2, there is shown a cardiopulmonary bypass vascular catheter assembly 10 including a left atrial vent catheter 12 and stylet 31.

Catheter 12 includes a flexible tube 16 which is preferably formed of an elastomer such as silicone rubber, so that the tube is soft and supple. Tube 16 is provided with a conical or radially outwardly flaring funnel connector 18 at the proximal end of the tube which receives one end 20 of a double-ended tube connector 22. The lumen 23 of the tube 16 is closed at the distal end by a catheter tip 24 having a smoothly rounded outer surface. Adjacent the distal end of tube 16 are a plurality of eyes or openings 26 extending through the sidewell of the tube 16. The funnel connector 18 may be formed of a suitable material such as silicone rubber and is connected to the tube 16 by an adhesive or bonding agent. The end 20 has tapered portions tapering inwardly in the distal direction and is connected to the funnel connectro 18 by a tight frictional engagement fit. The tip 24 is also preferably of silicone rubber and is fixed to the distal end of tube 16 by a suitable bonding agent, for example, a silicone adhesive. The tube connector 22 has a proximal end 28 which has portions tapering inwardly in the proximal direction and is adapted for frictional connection with tubing (not shown), such as tubing of an extracorporeal artificial heart-lung system. The connector 22 has a bore 30 extending through it which is in fluid communication with catheter lumen 23.

As seen also in Figure 3, the stylet 31 comprises an inner stylet rod or wire 32 extending within an outer covering 34 which is a separately formed sleeve. The proximal ends of the stylet wire 32 and the covering 34 are fixed to and within a stylet handle 36 which may be formed or moulded of a suitable plastic such as polyethylene or the like. The stylet wire 32 and covering 34 may be insert moulded in handle 36 during the moulding process. The stylet is shown as having a pair of crimps 38 with the proximal end of tube 16 extending over the distal crimp. The proximal end of covering 34 is thus closed to the atmosphere. A spherical distal end tip 40 is provided at the distal end of covering 34. The rounded tip 40 may be formed by melt forming the distal end of sleeve 34. Tip 40 closes the distal end of sleeve 34 to atmosphere and ensures that the distal end of wire 32 does not pierce the catheter 12. Thus, the entire wire 32 in the construction shown is completely enclosed by the covering 34 and handle 36, the covering completely enclosing the free surface of the wire while the handle covers the proximal end portion.

Handle 36 has a distal portion 42 which slidingly fits into the proximal end 28 of tube connector 22, and a proximally extending integral fixed arm 44. The handle has a pivotal latching arm 45 connected intermediate its ends to the arm 44 by an integral resilient connection 46. The arm 45 has a latch 47 at the distal end that co-operates with an integral annular flange 48 on the tune connector 22. Arm 45 has an end portion 49 extending proximally from the resilient connection 46. The stylet handle 36 is shown in Figures 1 and 4 in the latched condition with the latch 47 engaging the distal side of flange 48 to prevent proximal movement of the stylet 14 relative to the catheter 12. When in the latched condition, the stylet 14 extends to the distal region of catheter lumen 23 as shown in Figure 1. In this way, the stylet 14 is maintained in its desired fully inserted condition in catheter 12 so that the catheter and stylet can be inserted into a patient without the stylet moving longitudinally relative to the catheter and the distal end of catheter 12 bending excessively. When it is desired to remove the stylet from the catheter 12, the proximal end 49 of the latch arm 45 may be moved toward the arm 44, such as by pinching end 49 and arm 44, to unlatch the latch 47 from flange 48. This allows the stylet 14 to be withdrawn proximally from the catheter 12.

Stylet wire 32 is formed of a suitable malleable metal, such as aluminium or stainless steel, preferably, it is formed of solid aluminium. The covering 34 is preferably a plastic material and polypropylene is especially preferable because of its desirable characteristic of being readily slidable without undue force from its fully inserted condition as shown in Figures 1 and 4 to a fully removed condition (Figure 3). Covering 34 may be made of other materials such as high density polyethylene, polytetrafluoroethylene, fluorinated ethylene propylene or polyacetal in some catheters.

The stylet covering 34 should have an inner diameter sufficiently greater than the outer diameter of th wire (32) so as to allow insertion of wire (32) into the tubular cover means during assembly of the stylet. The covering may be a sleeve of plastic material such as an extruded sleeve of polypropylene. The diameter of the sleeve should be slightly larger than the wire to permit insertion of the wire into the sleeve during manufacture.

In use, with the stylet in place in catheter 12 as shown in Figure 1, the surgeon may manually curve or bend the assembly into a desired configuration for insertion, tip first, into the patient. Once catheter 12 is in its desired location, the stylet 31 is unlatched from connector 22, and withdrawn from catheter 12 and the tube connector 22 while maintaining the catheter tip 24 and openings 26 in position.

Catheter 12, being pliable and formed of a soft material, such as silicone rubber, is substantially inert to the body and there is less chance of damage to the patient than when catheters of more rigid materials are employed. The malleable stylet 31 can be readily bent by the surgeon into the desired shape with the pliable catheter tube 16 taking on any shape that is assumed by the stylet wire. Since the stylet 31 is capable of being easily deformed and maintains its deformity permanently or until reshaped, the supple catheter 12 surrounding the stylet is, of course, similarly deformed or shaped and remains deformed by the stylet. At the same time, the stylet 31 causes the assembly 10 to be sufficiently stiff so as to be inserted or worked into its desired final location within the patient without an undesirable amount of effort. As previously mentioned, the rounded tip 40 of stylet 31 aids in ensuring that the soft silicone rubber catheter tube 16 is not inadvertently pierced by the stylet wire 32 during typical insertion procedures. Since the stylet wire is completely enclosed by the covering 34 and the handle 36, blood cannot contact the metal wire.

It has been found that when the covering 34 is of polypropylene, the stylet readily slides on the silicone rubber sidewalls of lumen 23 substantially without sticking and even though the stylet and catheter may be curved or bent. This allows the stylet assembly 14 to be easily removed from catheter 14 while maintaining the catheter 12 in place within the patient. By employing the covering 34, the diameter of the stylet is effectively increased without increasing the size of wire 32 and thereby decreasing the malleability of the stylet. The stylet should be slightly less in diameter than the catheter lumen so that no undue effort is required for removal. By employing a stylet of relatively large diameter, such as stylet 31, good bending and insertion control characteristics with less flexing of the catheter relative to the stylet are obtained.

In the preferred embodiment a catheter and stylet assembly included a silicone rubber catheter having a lumen with a diameter of about 3.05mm and a stylet having an outer diameter of about 2.54mm. The stylet had a solid aluminium wire having an outer diameter of about 1.57mm and a covering sleeve cut from extruded polypropylene tubing having an inner diameter of about 1.70mm and an outer diameter of about 2.54mm, these being nominal values. The outer diameter of the wire is slightly less than the inner diameter of the covering to allow insertion of the wire into the covering during manufacture of the stylet. Thus, while the outer diameter of such wire was only about one-half of that of the catheter lumen, the overall outer diameter of the stylet, including the polypropylene covering, is about 83% of the diameter of the catheter lumen and provides good handling characteristics. About one-third of the diametral thickness of the stylet, is provided by the cover, and the cross-sectional area of cover is greater than that of wire. The above stylet may be used with a large catheter such as one having a lumen with a diameter of about 4.12mm. In this case, the diameter of the stylet is still more than one-half of that of the diameter of the larger catheter lumen and functions satisfactorily.

In the case of a left ventricular vent catheter, the catheter and stylet assembly can be made identical to assembly 10 except that the catheter holes will not generally be placed as far from the distal end of the catheter as they are in a left atrial vent catheter. Also, depending upon the use to which the catheter is to be put, the catheter tube material may be of a suitable thermoplastic polyurethane, latex rubber or the like instead of the preferred silicone rubber.

## Claims

1. A catheter and stylet assembly comprising a tube (16) of flexible material adapted for insertion into a patient and having a lumen (23) extending therein, and a stylet (31) assembly removably insertable into said lumen, the stytet including only one malleable metal wire (32), characterised by tubular cover means (34) surrounding and covering at least a major portion of said wire (32) including a portion adjacent the distal end of said wire, and a handle (36) connected to proximal end portions of said wire (32) and said tubular cover means (34), said tubular cover means (34) having an inner diameter sufficiently greater than the outer diameter of said wire (32) so as to allow insertion of said wire (32) into said tubular cover means (34) during assembly of said stylet.

2. An assembly as claimed in Claim 1 characterised in that said tube (16) is of an elastomeric material, and said cover means (34) is of a plastic material which is slidable relative to said tube (16) while in contact with said tube (16).

3. An assembly as claimed in Claim 1 or Claim 2 characterised in that said cover means is of polypropylene.

4. An assembly as claimed in any one of the preceding Claims characterised in that said wire is of aluminium.

5. An assembly as claimed in any one of the preceding Claims, characterised in that said cover means (34) includes a sleeve extending distally beyond the distal end of said wire and closed at its distal end to cover the distal end of said wire.

6. An assembly as claimed in any one of the preceding Claims characterised in that the proximal ends of said wire (32) and said cover means (34) are sealingly fixed within said handle (36) with said handle (36) and cover together completely enclosing said wire (32).

7. An assembly as claimed in anyone of the preceding Claims characterised in that the catheter is a cardiopulmonary bypass vascular catheter (10) of silicone rubber the lumen (23) of said catheter (10) is closed at the distal end, said catheter has a plurality of openings (26) adjacent the distal end extending through the sidewall thereof, and the stylet (31) extends substantially to the distal end of said lumen (23) when fully inserted therein, said tubular cover means is a tubular sleeve (34) of a plastic material covering substantially the entire outer surface of said wire (32), and the handle (36) is connected to the proximal end portions of said sleeve (34) and said wire (32), said tubular sleeve (34) having an inner diameter sufficiently greater than the outer diameter of said wire (32) so as to allow slidable insertion of said wire into said tubular sleeve (34) during assembly of said stylet and said sleeve (34) fixed with respect to said wire (32) by said handle (36)

8. An assembly as claimed in Claim 7 characterised in that said sleeve (34) is a tubular extrudate.

9. An assembly as claimed in any one of Claims 1 to 7, characterised in that said cover means (34) is a portion of a tubular plastic extrudate and wherein said wire (32) and said cover means (34) are generally straight and bendable.

10. An assembly as claimed in any one of Claims 1 - 9 characterised in that said handle (36) includes releasable latching means (47) for releasably securing said handle (36) to the proximal end portion of said tube with said stylet (31) extending in said tube.

**Patentansprüche**

1. Katheter- und Mandrin-Vorrichtung, mit einer Röhre (16) aus einem biegsamen Material, die zum Einführen in einen Patienten geeignet ist und ein Lumen (23) aufweist, und mit einem Mandrin (31), der in das Lumen herausnehmbar einführbar ist, wobei der Mandrin nur einen verformbaren Metalldraht (32) umfaßt, **gekennzeichnet durch**, röhrenförmige Überzugsmittel (34), die wenigstens einen Hauptteil des Drahtes (32) einschließlich eines an das distale Ende des Drahtes grenzenden Abschnitts umgeben und bedecken, und einen Halter (36), der mit den proximalen Endstücken des Drahtes (32) und der röhrenförmigen Überzugsmittel (34) verbunden ist, wobei die röhrenförmigen Überzugsmittel (34) einen Innendurchmesser aufweisen, der soviel größer ist als der Außendurchmesser des Drahtes (32), daß ein Einführen des Drahtes (32) in die röhrenförmigen Überzugsmittel (34) beim Zusammenbau des Mandrins möglich ist.

2. Vorrichtung nach Anspruch 1, dadurch gekennzeichnet, daß die Röhre (16) aus einem elastomeren Material besteht, und die Überzugsmittel (34) aus einem Kunststoffmaterial bestehen, das relativ zur Röhre (16) verschiebbar ist, obwohl es mit der Röhre (16) in Kontakt steht.

3. Vorrichtung nach Anspruch 1 oder 2, dadurch gekennzeichnet, daß die Überzugsmittel aus Polypropylen bestehen.

4. Vorrichtung nach einem der vorhergehenden Ansprüche, dadurch gekennzeichnet, daß der Draht aus Aluminium besteht.

5. Vorrichtung nach einem der vorhergehenden Ansprüche, dadurch gekennzeichnet, daß die Überzugsmittel (34) eine Hülse umfassen, die sich distal über das distale Ende des Drahtes hinaus erstreckt und an ihrem distalen Ende geschlossen ist, so daß sie das distale Ende des Drahtes bedeckt.

6. Vorrichtung nach einem der vorhergehenden Ansprüche, dadurch gekennzeichnet, daß die proximalen Enden des Drahtes (32) und der Überzugsmittel (34) in dem Halter (36) dicht mit dem Halter (36) verbunden sind und zusammen den Draht (32) vollständig umhüllend bedecken.

7. Vorrichtung nach einem der vorhergehenden Ansprüche, dadurch gekennzeichnet, daß der Katheter ein Gefäßkatheter (10) für einen cardiopulmonalen Bypass aus Silikonkautschuk ist, das Lumen (23) des Katheters (10) am distalen Ende geschlossen ist, wobei der Katheter eine Vielzahl von Öffnungen (26) aufweist, die dem distalen Ende benachbart sind und durch dessen Seitenwand führen, der Mandrin (31) sich im wesentlichen bis zum distalen Ende des Lumens (23) erstreckt, wenn er vollständig in dieses eingeschoben ist, die röhrenförmigen Überzugsmittel eine röhrenförmige Hülse (34) aus einem Kunststoffmaterial darstellen, die im wesentlichen die gesamte äußere Oberfläche des Drahtes (32) bedeckt und der Halter (36) mit den proximalen Endstücken der Hülse (34) und des Drahts (32) verbunden ist, wobei die röhrenförmige Hülse (34) einen Innendurchmesser aufweist, der soviel größer ist als der Außendurchmesser des Drahtes (32), daß ein gleitendes Einführen des Drahtes in die röhrenförmige Hülse (34) beim Zusammenbau des Mandrins möglich ist, und wobei die Hülse (34) mit Hilfe des Halters (36) gegenüber dem Draht (32) fixiert wird.

8. Vorrichtung nach Anspruch 7, dadurch gekennzeichnet, daß die Hülse (34) ein röhrenförmiges Extrudat ist.

9. Vorrichtung nach einem der Ansprüche 1 bis 7, dadurch gekennzeichnet, daß die Überzugsmittel (34) ein Teilstück eines röhrenförmigen Kunststoff-Extrudats sind und der Draht (32) und die Überzugsmittel (34) im allgemeinen gestreckt und biegsam sind.

10. Vorrichtung nach einem der Ansprüche 1 bis 9, dadurch gekennzeichnet, daß der Halter (36) lösbare Einrastmittel (47) zum lösbaren Befestigen des Halters (36) an dem proximalen Endstück der Röhre aufweist, wobei der Mandrin (31) in der Röhre ist.

## Revendications

1. Ensemble de cathéter et de stylet comprenant un tube (16) en matière flexible propre à être introduit dans un patient et comportant un passage intérieur (23) qui s'étend dans ce tube, et un ensemble de stylet (31) pouvant être introduit dans le passage, le stylet comprenant un seul fil métallique malléable (32), caractérisé par une enveloppe tubulaire (34) entourant et recouvrant au moins une partie majeure du fil (32) comprenant une partie adjacente à l'extrémité distale du fil, et une poignée (36) reliée aux parties d'extrémité proximales du fil (32) et de l'enveloppe tubulaire (34), l'enveloppe tubulaire (34) ayant un diamètre intérieur suffisamment supérieur au diamètre extérieur du fil (32) pour permettre l'introduction du fil (32) dans l'enveloppe tubulaire (34) pendant le montage du stylet.

2. Ensemble suivant la revendication 1, caractérisé en ce que le tube (16) est fait d'une matière élastomère et l'enveloppe (34) est faite d'une matière plastique qui peut coulisser par rapport au tube (16) tandis qu'elle se trouve en contact avec le tube (16).

3. Ensemble suivant la revendication 1 ou 2, caractérisé en ce que l'enveloppe est en polypropylène.

4. Ensemble suivant l'une quelconque des revendications précédentes, caractérisé en ce que le fil est en aluminium.

5. Ensemble suivant l'une quelconque des revendications précédentes, caractérisé en ce que l'enveloppe (34) comprend une gaine qui s'étend dans la direction distale au-delà de l'extrémité distale du fil et qui est fermée à son extrémité distale pour couvrir l'extrémité distale du fil.

6. Ensemble suivant l'une quelconque des revendications précédentes, caractérisé en ce que les extrémités proximales du fil (32) et de l'enveloppe (34) sont fixées d'une manière étanche dans la poignée (36), la poignée (36) et l'enveloppe enfermant ensemble complètement le fil (32).

7. Ensemble suivant l'une quelconque des revendications précédentes, caractérisé en ce que le cathéter est un cathéter vasculaire de dérivation cardiopulmonaire (10) en caoutchouc de silicone, le passage (23) du cathéter (10) est fermé à son extrémité distale, le cathéter présente une pluralité de lumières (26) à proximité de son extrémité distale qui traversent sa paroi latérale, et le stylet (31) s'étend en substance jusqu'à l'extrémité distale du passage (23) lorsqu'il y est introduit à fond, l'enveloppe tubulaire est une gaine tubulaire (34) en une matière plastique recouvrant en substance la totalité de la surface extérieure du fil (32), et la poignée (36) est reliée aux parties d'extrémité proximales de la gaine (34) et du fil (32), la gaine tubulaire (34) ayant un diamètre intérieur suffisamment supérieur au diamètre extérieur du fil (32) pour permettre l'introduction à coulissement du fil dans la gaine tubulaire (34) pendant le montage du stylet et de la gaine (34) fixée par rapport au fil (32) par la poignée (36).

8. Ensemble suivant la revendication 7, caractérisé en ce que la gaine (34) est un élément tubulaire extrudé.

9. Ensemble suivant l'une quelconque des revendications 1 à 7, caractérisé en ce que l'enveloppe (34) est une partie d'un élément extrudé tubulaire en matière plastique et le fil (32) et l'enveloppe (34) sont généralement droits et pliables.

10. Ensemble suivant l'une quelconque des revendications 1 à 9, caractérisé en ce que la poignée (36) comprend un moyen de verrouillage libérable (47) destiné à fixer de manière libérable la poignée (36) à la partie d'extrémité proximale du tube, le stylet (31) s'étendant dans le tube.

FIG. 1

FIG. 3

FIG. 2

FIG. 4

EP 0 242 985 B1